# EUROPEAN PATENT SPECIFICATION

(11) **EP 2 579 808 B1**
(45) Date of publication and mention of the grant of the patent: **14.09.2022**
(21) Application number: 11793135.2
(22) Date of filing: 09.06.2011
(51) Int. Cl.: A61F 2/00, A61B 17/00

(54) **ADHESION-RESISTANT SURGICAL ACCESS, REINFORCEMENT AND CLOSURE PROSTHETIC**
KLEBERESISTENTE CHIRURGISCHE ZUGANGS-, VERSTÄRKUNGS- UND VERSCHLUSSPROTHESE
ACCÈS CHIRURGICAL RÉSISTANT À L'ADHÉSION, RENFORCEMENT ET PROTHÈSE DE FERMETURE

(30) Priority: 09.06.2010 US 353156 P; 15.06.2010 US 355053 P
(43) Date of publication of application: 17.04.2013
(73) Proprietor: MAST Biosurgery AG, 8002 Zürich (CH)
(72) Inventor: BLUECHER, Lukas, D82547 Eurasburg (DE); MILBOCKER, Michael, T., Holliston, MA 01746 (US); CALHOUN, Christopher, J., San Diego, CA 92130 (US)
(74) Representative: Potter Clarkson
(86) International application number: PCT/US2011/039721
(87) International publication number: WO 2011/156552

(56) References cited:
- WO-A1-03/007847
- WO-A1-2009/131682
- WO-A2-2009/156866
- WO-A2-2010/039978
- GB-A- 2 406 522
- US-A- 4 259 959
- US-A1- 2003 195 607
- US-A1- 2004 267 362
- US-A1- 2005 165 447
- US-A1- 2006 287 719
- US-A1- 2008 177 253
- US-A1- 2008 208 359
- US-A1- 2009 010 983
- US-A1- 2009 010 983
- US-A1- 2009 304 779
- US-A1- 2010 023 132
- US-A1- 2010 114 328
- US-B1- 6 383 201
- US-B1- 6 451 032

## Description

### BACKGROUND OF THE INVENTION

### 1. Field of the Invention

The present invention relates generally to medical implants. In particular, a composite membrane prosthetic is disclosed suitable for replacement of the parietal pericardium subsequent to heart surgery.

### 2. Description of Related Art

Wound healing involves the formation of adhesions between tissue surfaces, typically comprising fibrotic tissue, and these adhesions are characteristic of the initial phases of tissue regeneration. The fibrotic response to tissue damage is non-specific to tissue micro-structure and does not respect boundaries between normally mechanically independent tissue surfaces. This aspect of the tissue repair mechanism presents a special problem for surgical techniques that require access to tissue enclosed in living tissue membranes meant to separate dynamic tissue surfaces, such as the function of the pericardium in separating the beating heart from the lungs, surrounding vasculature and bone. Therefore, there is a need to provide an access and repair prosthetic which provides closure after access, restores the natural barrier properties of the breached tissue, and provides a conductive matrix in which to channel and direct tissue repair as is normally initiated whenever tissue is damaged.

The healing process subsequent to surgery is non-specific in its locus of activity and tends to generate excessive or clinically unacceptable amounts of scar tissue, or fibrosis. Scar tissue can in several instances block arteries, immobilize joints, damage internal organs, and can in some instances generally or partially impede a body's ability to maintain vital functions. Every year, about 1.3 million people are hospitalized due to the damaging effects of fibrosis. Excessive fibrosis takes several forms: organ-to-organ adhesions, keloid tumors or hypertrophic (very severe) scarring, and growth that invade the surrounding healthy tissue, even after the original injury heals. Too much scar tissue may cause physiological roadblocks that in severe cases can disfigure, cripple or even kill.

An important pathology in which fibrosis can be particularly problematic is cardiac surgery. The number of patients undergoing cardiac surgery has been steadily increasing, and as a consequence, the number of cardiac complications has also increased. One of the more common forms of adhesions in this context can occur after the cardiac surgery as a direct result of the current or a prior cardiac surgical intervention.

Cardiac surgical procedures can often be associated with unique conditions of adhesion, which can begin to form immediately following the surgical procedure. Such conditions can persist often times with lower rates of treatment success as compared to similar complications of other tissues. Despite the need for effective treatments, doctors typically have few optimal remedies to help them control this relatively prevalent condition.

In addition to being relatively prevalent, such conditions have proven themselves to also be relatively persistent. That is, in the context of cardiac surgical procedures, scar tissue treatments tend to be less effective in preventing or attenuating fibrosis than with other procedures.

For example, thin-membrane implantation measures for attenuating adhesion following cardiac surgery, such as disclosed in U.S. Application No. 10/660,461, tend to be less effective than similar procedures applied to other tissues and/or other parts of the body. The continuously flexing and differential motion of cardiac tissue relative to the surrounding tissue presents a characteristic case where the motion contributes to the continued release of cytokines that tend to promote a chronic inflammatory response. In addition, all fibrotic repairs, by virtue of their relatively acellular composition, tend to be remodeled by cellular processes over time. Wherever the cellular environment tends to remain in a transitional state, the region affected by fibrotic repair processes increases, and tissues with specialized functions tend to be compromised by the indiscriminate joining these processes precipitate. In an attempt to direct the diffuse nature of the healing process in a cardiac surgical procedure, for example, surgeons may place a thin resorbable membrane over one or more vicinities of tissue affected by a procedure (e.g., over one or more areas, apertures, protuberances, or edges, which were created by or were affected by an incision). Nevertheless, these approaches tend to be less effective in providing an optimum or even adequate attenuation of tissue adhesions in that vicinity, again potentially as a result of a relatively excessive amount, or nature, of movements of or near the affected tissue in need of treatment. In particular, while such membranes provide temporary barriers to adhesion formation, they do not direct the healing process toward repair of the tissue, and consequently the non-specific scar tissue that forms becomes the source of continued irritation, remodeling, and release of cytokines that promote adhesion, sometimes long after the surgical barrier has been resorbed or encapsulated.

There continues to be a need for improved methods of inducing proper tissue healing, e.g. healing without adhesion or with reduced rates of adhesion, for relatively non-static tissues affected by surgical interventions such as open heart surgery.

Heretofore, a number of patents and publications have disclosed devices for closing surgical wounds to aid in healing and to prevent post-surgical adhesions, the relevant portions of which may be briefly summarized as follows:
U.S. Patent No. 5,679,112 describes calcification-resistant materials and methods of making same through use of multivalent cations in which bioprosthetic materials, either natural or synthetic, are treated with trivalent iron cations, or salts, to prevent in vivo calcification.
U.S. Patent No. 5,084,101 describes a composition for the production of an open-pore foam product from essentially inorganic components containing, in addition to known components, a rock-forming solid substance, a curing agent, a foaming additive, and an at least partly alkali-soluble protein to produce open pores.
U.S. Patent No. 5,906,997 discloses bioresorbable compositions of carboxypolysaccharide polyether intermacromolecular complexes and methods for their use in reducing surgical adhesions wherein improved methods for making and using bioadhesive, bioresorbable, anti-adhesion compositions made of intermacromolecular complexes of carboxyl-containing polysaccharides and polyethers, and to the resulting compositions are described.
U.S. Patent No. 6,150,581 relates to chitosan/alginate post-surgical anti-adhesion barriers, methods of preventing post-surgical adhesions, and methods and devices for forming post-surgical anti-adhesion barriers are provided.
U.S. Patent No. 6,251,419 describes a membrane system for controlled tissue regeneration of the periodontium, comprising a resorbable polymer membrane and anti-adhesion molecules.
U.S. Patent No. 6,287,250 discloses a clip which can be temporarily clamped to a cut edge of a pericardium during heart surgery. Two or more of the clips, which may be each connected to a respective flexible member, are clipped to the pericardium and tension is applied to the connectors to suspend the clips and the pericardium, as well as the heart, form a support, while enabling the heart to continue to beat normally.
U.S. Patent No. 6,383,201 discloses a surgical prosthesis for repairing a hernia in a sturdy tension-free manner. The prosthesis includes a layer of adhesion resistant material and a first layer of tissue ingrowth receptive mesh material affixed to the layer of adhesion resistant material. A second layer of tissue ingrowth receptive mesh material is positioned adjacent the first layer of tissue ingrowth receptive mesh material and a connecting thread is interwoven between the midsections of the layers for forming a mid-sectional seam fastening together the three layers.
U.S. Patent No. 6,440,167 relates to a collagen material comprising a laminate in which a collagen ultra-fine fibrous non-woven fabric-like multi-layer structure is sandwiched between non-fibrous collagen layers, a thread-like material containing said collagen material, their production processes, and a medical material containing said collagen material, and particularly a medical alternative membrane comprised of said medical material.
U.S. Patent No. 6,451,032 describes a composite prosthesis comprising a prosthetic fabric having a three-dimensional structure separating two surfaces, one of which is open to post-surgical cell colonization. The composite prosthesis further comprises at least one film of a collagenous material linked to the other surface of the prosthetic fabric. The composite prosthesis may be used to prevent post-surgical adhesion.
U.S. Patent No. 6,596,304 discloses a biocomposite material based on collagen prepared with two closely bound layers, and is biocompatible, non-toxic, hemostatic and biodegradable in less than a month, and can be used in surgery to achieve hemostasis and prevent post-surgical adhesion.
U.S. Patent No. 6,599,526 discloses a device directed to an anti-adhesion patch, which is constructed using a tissue equivalent technique. The anti-adhesion patch comprises a collagenous material and at least one non-living cellular component. Also provided is a method for preventing tissue adhesions between organs and other tissues being operated upon during surgical procedures by utilizing the anti-adhesion patch disclosed herein.
U.S. Patent No. 6,652,559 discloses a wound closure system for closing a wound on a patient comprising an elongated flexible backing strip having a length and width sufficient to secure facing edges of the wound in close juxtaposition to one another, the backing strip comprising a first portion disposed between the ends and adapted to overlie the facing edges of the wound, and second and third portions disposed on either side of the first portion and each provided with a predetermined number of spaced-apart apertures extending through the backing strip from one surface thereof to the other; and a first pressure-sensitive adhesive coated on at least part of the first surface of the backing strip including the second and third portions thereof, to adhere the backing strip to the patient with the facing edges of the wound being in close juxtaposition,
U.S. Patent No. 6,693,089 discloses a method of reducing adhesion at a site of trauma including forming a film from an alginate solution, contacting the film with a cross-linking solution to form a cross-linked mechanically stable sheet, and placing at least a portion of the sheet at the site of trauma. An anti-adhesion barrier includes a sheet of ionically cross-linked alginate having a thickness in a range of 0.25 mm to 10 mm.
U.S. Patent No. 6,743,521 discloses compositions for coating biological and nonbiological surfaces, which minimize or prevent cell-cell contact and tissue adhesion, and methods of preparation and use. Embodiments include polyethylene glycol/polylysine (PEG/PLL) block or comb-type copolymers with high molecular weight PLL (greater than 1000, more preferably greater than 100,000); PEG/PLL copolymers in which the PLL is a dendrimer which is attached to one end of the PEG; and multilayer compositions including alternating layers of polycationic and polyanionic materials. The multi-layer polymeric material is formed by the ionic interactions of a polycation and a polyanion. The molecular weights of the individual materials are selected such that the PEG portion of the copolymer inhibits cellular interactions, and the PLL portion adheres well to tissues.
U.S. Patent No. 6,869,938 discloses an invention that relates to improved methods for making and using bioadhesive, bioresorbable, anti-adhesion compositions made of intermacromolecular complexes of carboxyl-containing polysaccharides, polyethers, polyacids, polyalkylene oxides, multivalent cations and/or polycations. The polymers are associated with each other, and are then either dried into membranes or sponges, or are used as fluids or microspheres.
U.S. Patent No. 7,060,023 discloses a device for reinforcing the pericardial sac surrounding the heart to assist in the treatment of congestive heart failure. The device, generically, is an enclosure having an interior and an exterior. The interior surface is made in such a way that it tends not to or does not form adhesions with or accept ingrowth with the myocardial tissue of the epicardium. The exterior surface of the device, in contrast, is adapted to adhere to or to ingrow with or otherwise attach sufficiently to the pericardium so that it reinforces that membrane or structure. The nature of the device is that it tends not to allow the pericardium to expand further with time. The device, after complete deployment, should envelope some measure of pericardial fluid in its interior separating it from the epicardial surface.
U.S. Patent No. 7,144,588 relates to a method and composition for preventing surgical adhesions during surgery. Tissue surfaces and/or surgical articles involved in the surgery are separated by a biomaterial provided in the form of a non-crosslinked, decellularized and purified mammalian tissue (e.g. bovine pericardium). The biomaterial effectively inhibits fibrosis, scar formation, and surgical adhesions, while also serving as a scaffold for recellularization of the tissue site.
U.S. Patent No. 7,491,408 describes a novel polymer having a number average molecular weight of 10000 to 100000 that has excellent bioabsorbability so as to disappear in a predetermined period of time even when embedded in a large amount in the body, has good kinetic properties, and is useful for preparing tissue anti-adhesion films.
U.S. Patent No. 7,618,410 describes a method of accessing the pericardial space comprising providing a primary catheter having an inflatable balloon, inserting the primary catheter into the vascular system, forwarding the primary catheter through the vascular system to an atrial appendage, inflating the balloon to engage the wall of the appendage sufficient to separate opposing wall portions of the appendage between the cardiac tissue and the pericardium.
U.S. Patent No. 7,641,682 discloses an implantable medical graft fabricated of metallic or pseudometallic films of biocompatible materials having a plurality of microperforations passing through the film in a pattern that imparts fabric-like qualities to the graft or permits the geometric deformation of the graft.
U.S. Patent No. 7,704,520 describes a resorbable polylactide polymer healing membranes and methods of their applications are disclosed. In a broad embodiment, the invention features methods for inducing proper tissue healing after an open heart surgery. In one embodiment, the methods includes a step of forming a patch with a healing membrane over the open pericardium to induce proper tissue healing and placement in other open heart surgery procedures to facilitate re-entry by the surgeon.
U.S. Patent No. RE39,172 describes a biocomposite material based on collagen which has two closely bound layers and is biocompatible, non-toxic, hemostatic and biodegradable in less than a month, and can be used in surgery to achieve hemostasis and prevent post-surgical adhesion.
U.S. Application No. 2009/0291925 discloses bioactive liquid formulations formed of combinations of absorbable, segmented aliphatic poiyurethane compositions and liquid polyether for use as vehicles for the controlled release of at least one active agent for the conventional and unconventional treatment of different forms of cancer and the management of at least one type of bacterial, fungal, and viral infection.
U.S. Application No. 2009/0192554 discloses a bioabsorbable poiyurethane block copolymer with having hard and soft segments is formed by the reaction between a diisocyanate such as lysine ethyl ester diisocyanate and a mixture of (i) an aromatic amide or an amino acid derivative and (ii) a polyether glycol and/or an absorbable elastomeric polymer derived from the reaction between dialkylene glycol and a cyclic bioabsorbable monomer such as trimethylene carbonate, caprolactone, or dioxanone.
Canadian Patent No. 2,580,624 describes a preparation for biocompatible, optionally biodegradable open-cell poiyurethane foam having open pores also at its surface.
WO 03/007847 describes devices and methods for reinforcing a layer of living tissue, which when affixed to a tissue layer prior to surgical incision, reinforces the tissue to be incised, provides a fibro-conductive matrix to promote healing in a preferred plane, and provides for a subsequent closure and fluidic seal. A partially or entirely absorbable growth matrix is disclosed, comprising two adhesion-resistant layers enclosing a cellular conductive medium for promoting fibrosis in a preferred plane. The cellular conductive portion is partially or entirely sequestered from surrounding tissue. The device is constructed in a physiologic range of tensile strengths and elasticity suitable for closure of the pericardium, peritoneum, or other typically thin membranes enclosing organs in the body, whose function is to prevent adhesions between tissue surfaces normally in motion.

Most anti-adhesion surgical barriers in use today are passive patches, meant to provide a sealing layer after the functional aspect of the surgery has been performed. However, much of the fibrotic potential of a procedure can be lessened if the surgical barrier itself facilitates access and closure of the affected tissue. It is therefore an object of the present invention to provide a composite structure which can be positioned on tissue prior to surgical invasion, which localizes tissue damage and provides for tissue closure that minimizes localized mechanical aspects, such as stress, that promote cellular death.

While most anti-adhesion barriers are designed to be absorbable, the very nature of absorbable implants creates a dynamic architecture which itself promotes fibrosis. It is therefore an object of the present invention to pro vide a medium in which to channel this growth response, which is designed to repair the primary insult and minimize the degree of chronic disruption of the tissue surrounding the repair before the absorbable barrier disappears.

Even when the tissue growth is directed, it is equally important that the new growth mimic natural tissue as closely as practicable. One of the chief differentiating features of living tissue over scar tissue is the abundance of cells and the needed vascular structures to provide oxygen and nutrients to those cells.

It is also important to recognize that nearly all surgical repairs result in the implantation of foreign bodies such as mesh and suture, including the anti-adhesion layer itself. As foreign bodies, they alter intra-cellular chemistry in such a way that they precipitate a chemotactic response causing the release of reactive oxygen species and the accumulation of inflammatory cells. Thus any cellular response exterior to the healing region is minimized by both a physical barrier and also the elimination of the need for a cellular response exterior to the healing region. In surgical repairs, the necessity for breaching boundary tissue layers is often overlooked and their importance under appreciated. These layers are typically very thin, such as the dura that encircles the nervous system, the pericardium that encircles the heart, the various tissues encircling all conduits of blood and the peritoneum that encircles the active portions of the digestive tract. They are by their dimensional and mechanical aspects difficult to identify and repair, and repairs if achieved, compromise their barrier functionality. They are particularly susceptible to stress, strain, and very slight abrasion. In particular, attachment, which is necessary for dispersing localized stress induced by apposing tissue surfaces in a repair is likewise non-local, and provided before the surgical incision is made. Many of these tissues undergo contraction when incised, and the edges of the incised tissue cannot be brought back in apposition in a repair procedure in a tension-free manner,

### SUMMARY OF THE INVENTION

According to the present invention there is provided a prosthetic for prompting healing of damaged tissue after an open heart surgery according to claim 1.

An adhesion-resistant surgical access, reinforcement and closure prosthetic (closure prosthetic) may assist the healing function of a human tissue disposed within a body and comprising a plurality of outer layers sandwiching an inner layer which may be porous and conductive to both chemical and cellular infiltrates. The sandwiching layers may resist tissue attachment, be comprised of a bio-resorbable polymer, such as poly-lactic acid or certain polyurethanes, and possess a maximum thickness suitable for insertion into the body via laparoscopic means. Optimally, there are two sandwiching layers defining proximal and distal surfaces that mimic the proximal and distal surfaces of the original tissue on which a surgical incision is intended. Between the sandwiching layers may be disposed a tissue scaffold which is oriented such that the edges of the incision made by a surgical procedure preferentially grow together in a normal anatomical aspect when the prosthetic is implanted to repair such a surgical incision. Preferably, the porosity is interconnected and provides for tissue growth throughout its volume such that two tissue edges meant to be joined can grow into the scaffold layer and eventually meet within the prosthetic. The scaffold layer possessing a porosity sufficient to permit small vessels to grow throughout it, of preformed shape or substantially planar, having an exterior wall, an interior wall, and a tissue scaffold juxtaposed between them, and an equatorial margin where the outer layers are present but the tissue scaffold is not, with an outer surface and an inner surface capable of sandwiching tissue, an inner scaffold medium joined to said exterior wails so as to form a volume between said equatorial margin; and the inner medium preferably in contact with said entrapped tissue such that first fluids and then ceils are conducted throughout the inner medium.

A method for assisting the healing function of a human tissue disposed within a body, including outer walls and cell conductive medium may utilize a first attachment means; an incision means; an inner wall unfolding means such that when the prosthetic is applied to a target tissue layer, this layer having proximal and distal sides, the prosthetic affixes to the distal surface of said tissue layer, an incision is made through the prosthetic and affixed tissue layer and the second inner layer of the prosthetic deploys onto the proximal side of said tissue layer, and a means for closing said formed incision.

A method for assisting the healing function of a human tissue disposed within a body, including outer wails and cell conductive medium may utilize a first attachment means; an incision means; an inner wall unfolding means such that the inner wail is folded across a central portion of the prosthetic such that said equatorial margin consists only of the distal prosthetic layer. When the prosthetic is applied to a target tissue layer, this layer having proximal and distal sides, the prosthetic affixes to the distal surface of said tissue layer such that a lifting force can be applied to the prosthetic and the tissue layer lifted away from adjacent tissue such that an incision can be made without endangering said adjacent tissue. The prosthetic may include a preformed slit or plurality of slits, some of which may be orthogonal to others at a select location so that the incision may be made directly to said tissue layer and the inner layer of the prosthetic made to engage the proximal surface of said tissue layer by folding into the void thus created and a means for closing said formed incision.

The closure prosthetic described herein is advantageous because compared to other anti-adhesion devices, it utilizes tissue scaffolding to conduct the healing power of the body without allowing adjacent tissue to adhere thereof, or the cellular contents released by the incision to cause fibrosis away from the healing site. In a preferred embodiment the closure prosthetic uses at least one attachment means to reinforce the prosthetic-tissue prior to the placement of a surgical incision an d a second attachment means capable of sandwiching the tissue edges of the incision at the moment the incision is executed permitting the incision to be spread open by a simple procedure that can be quickly performed without damaging said sandwiched tissue. In addition, the closure device localizes the wounded tissue so as to minimize proliferation of fibrosis and focuses the healing response to the wound site. The closure prosthetic mimics the functional performance of the tissue prior to incision once it is closed after the incision i s made, which minimizes repeated re-injury of the healing site. Repeated re-injury of a wound site is an important etiology for chronic inflammation, excessive scarring, and adhesion to adjacent tissues. This ultimately generates a faster repair, and hastens the onset of tissue quiescence, or resumption of normal cytokine levels. As a result, a greater variety of surgical procedures can be provided with minimal post-operative pain under a greater variety of circumstances, including but not limited to, enhanced capacity for tissue healing, restoration of pre-incisional function, and enhanced or even permanent functionality because it utilizes a novel combination of wound healing stimulus in combination with sequestration of the wound healing process.

### BRIEF DESCRIPTION OF THE DRAWINGS

The invention will be described by reference to the following drawings, in which like numerals refer to like elements, and in which:
FIG. 1 illustrates one embodiment of the closure prosthetic of the present invention comprised of three planar layers.
FIG. 2 illustrates one embodiment of the closure prosthetic when engaged with tissue.
FIG. 3A illustrates the localization mechanism of the closure prosthetic.
FIG. 3B illustrates the tissue capture mechanism involving folding the proximal layer of the closure prosthetic.
FIG. 3C illustrates the tissue sandwiching mechanism of the closure prosthetic.
FIG. 3D illustrates the sealing mechanism of the closure prosthetic.
FIG. 4A illustrates the localization mechanism of a folded embodiment of the closure prosthetic,
FIG. 4B illustrates the folding mechanism engaged in the capture and sealing of a tissue defect with the closure prosthetic.
FIG. 5 illustrates the localization and capture mechanism of a toggled folded embodiment of the closure prosthetic.
FIG. 6 illustrates a two-part closure prosthetic useful in open-heart surgery.
FIG. 7 illustrates the pericardial hammock configuration of the closure prosthetic in an open sternotomy presentation.
FIG. 8 illustrates another two-part closure prosthetic employing snares.
FIG. 9 illustrates a closure prosthetic for correcting aneurysm of a vessel,
FIG. 10 illustrates a closure prosthetic useful in accessing the cardiac atrium.
FIG. 11A illustrates a closure prosthetic useful in reinforcing a bypass anastomosis.
FIG. 11B illustrates a closure prosthetic engaging a bypass anastomoses.
FIG. 12 illustrates a three-layer closure prosthetic for repair of the parietal pericardium.
FIG. 13 illustrates a closure prosthetic of the present invention relative to its placement on a human heart.

### DETAILED DESCRIPTION OF THE INVENTION

In the drawings, like reference numerals have been used throughout to designate identical elements.

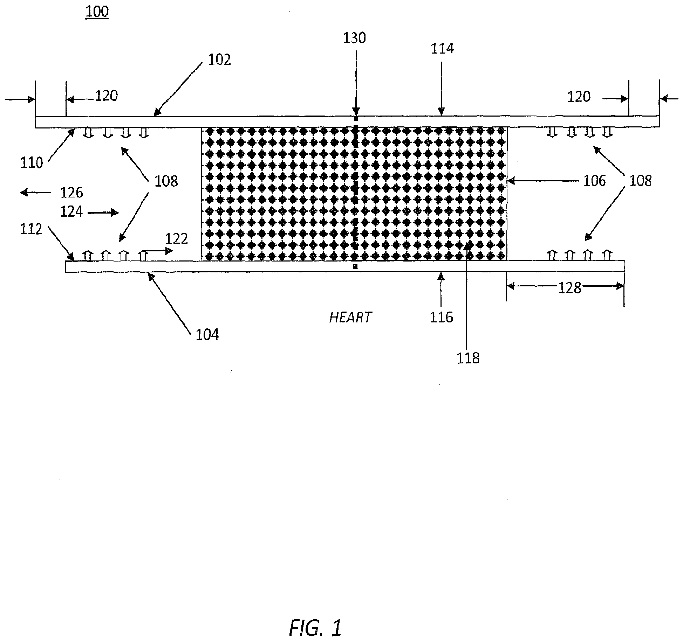

Preferred devices and methods invention will now be described in detail, with reference to FIGS. 1-6. This description will begin with particular embodiments of the closure prosthetic, then attention will be directed to description of particular materials of construction, a detailed description of particular methods of implantation of the closure prosthetic, and finally novel features will be described with respect to their benefit and clinical utility.

### Closure Prosthetic, Example 1

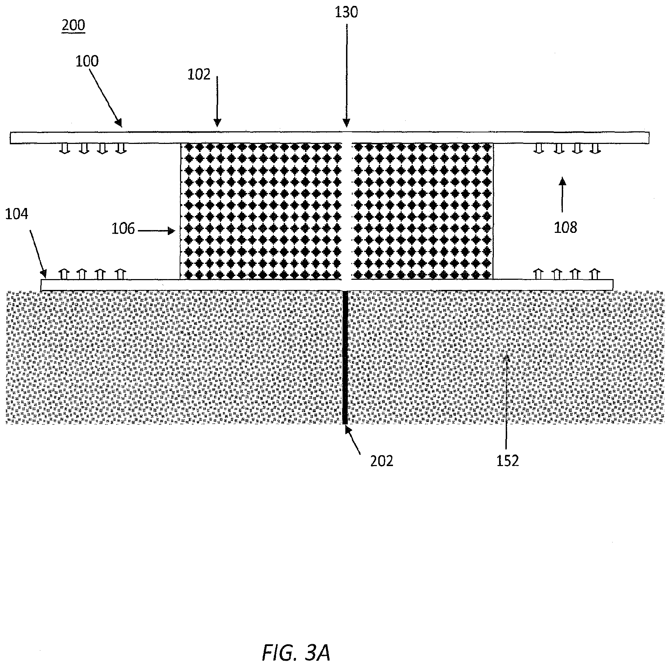

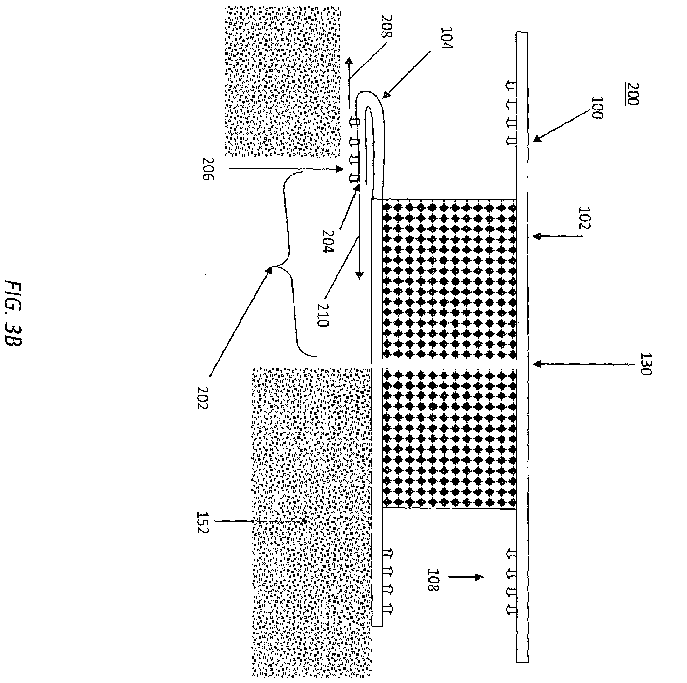

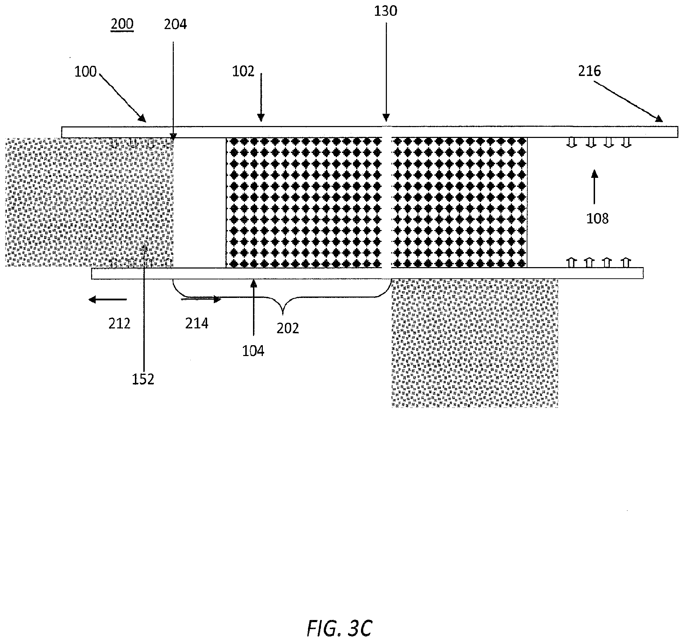

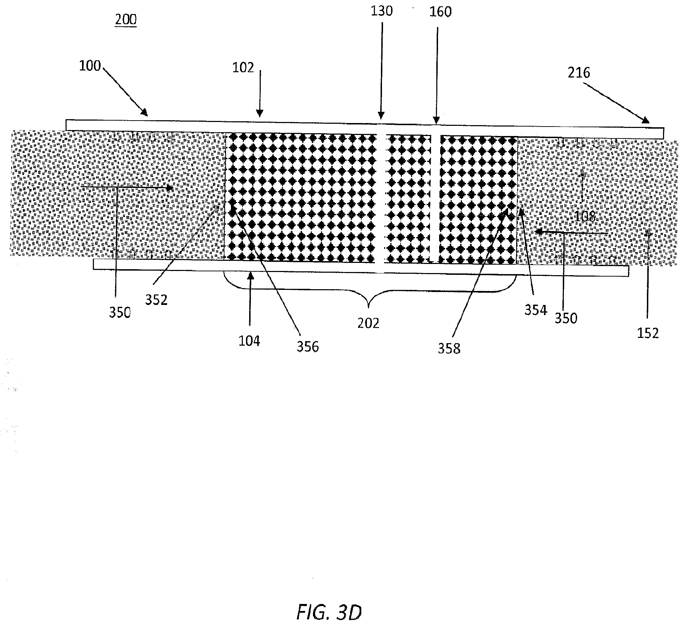

Referring to FIG. 1, a closure prosthetic 100 is comprised of an absorbable distal layer 102, an absorbable proximal layer 104, a sandwiched tissue scaffold layer 106, and attachment means 108. The attachment means 108 are located on distal interior side 110 of layer 102 and proximal interior side 112 of layer 104. Layers 102 and 104 have exterior distal sides 114 and exterior proximal sides 116, respectively. Sides 114 and 116 are smooth and resist tissue attachment. Layers 102 and 104 are bonded to opposite sides of tissue scaffold layer 106. Tissue scaffold layer 106 is a porous, flexible medium. The pores 118 are interconnected and sufficiently large and numerous to allow cells to infiltrate and tissue to grow into the tissue scaffold layer 106. Distal layer 102 is slightly larger than proximal layer 104, and both layers 102 and 104 are larger than tissue scaffold layer 106. The layers are bonded together in this arrangement, thereby defining a margin 120. Margin 120 allows for the closure prosthetic to be localized on a tissue surface to be incised. Margin 120 is defined by the excess area of distal layer 102 relative to the area of proximal layer 104. On the entire interior side 110 of layer 102 are located the attachment means 108. Attachment means 108 have an orientation 122 such that tissue slides easily against the attachment means 108 in direction 124 but is prevented from slipping and is engaged in direction 126. Similarly, attachment means 108 are located on the proximal interior side 112 of layer 104. A second margin area 128 is defined on distal layer 102 and proximal layer 104 and includes the area of each not occupied by tissue scaffold layer 106 or margin 120. The working surfaces of margin area 128 correspond to the interior surfaces of layers 102 and 104 where attachment means 108 are disposed. The function of margin area 128 is to sandwich the free ends of tissue formed by making an incision, as will be more fully described in the description of FIG. 3, below. Additional features include optionally a slit 130 which provides access to underlying tissue to which an incision may be made.

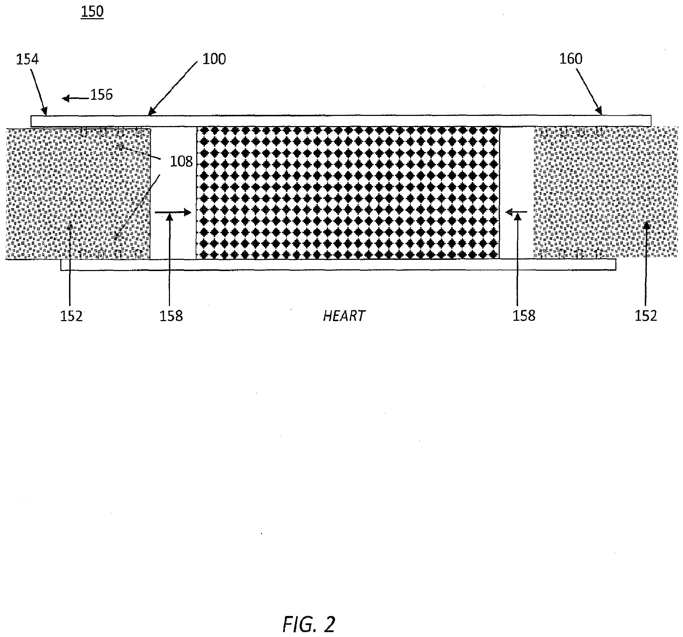

Referring now to FIG. 2, the first function 150 of closure prosthetic 1 00 is illustrated. The closure prosthetic is anchored to a tissue layer 152 by means of attachments 108. Light pressure is sufficient to engage the tissue layer 152 to the closure prosthetic 100. Alternatively, an edge 154 is placed in contact with tissue layer 152 and the closure prosthetic pulled in direction 156 to engage attachments means 108 situated along contact region 154. Tissue layer 152 resists motion in direction 156 such that the tissue layer 152 places tension on the closure prosthetic 100 in direction 158. When opposite edge 160 is placed in contact with tissue layer 152 the attachment means 108 fix to tissue layer 152 as a result of tension in the closure prosthetic 100 in direction 158.

Referring now to FIG. 3A, the second function 200 of closure prosthetic 100 is illustrated. The closure prosthetic is anchored to a tissue layer 152 by means of attachments 108. Access through tissue layer 152 is desired. A scalpel (not pictured) is placed in slit 130 and incision 202 is made. Note slit 130 transects distal layer 102, proximal layer 104 and tissue scaffold layer 106. Alternatively, slit 130 is absent and the surgeon produces such a slit in closure prosthetic 100 with a scalpel. Once the slit is made, there is a need to retract the tissue layer 152 to widen incision 202. This is done by grasping one side 204 of proximal layer 104 with a surgical grasping tool (not pictured), folding it back upon itself, and passing it through incision 202, as illustrated in FIG. 3B. The attachment means 108 disposed on proximal layer 104 on side 206 grasp tissue in direction 208, thus inverting proximal layer 104 and exposing side 206 to tissue layer 152 orients the grasping direction 208 counter to sliding direction 210 and consequently attachment means 108 do not engage tissue. Referring now to FIG. 3C, Passing edge 204 through incision 202 reverses the sliding direction 212 and also the grasping direction 214 so that edge 204 returns to its former position but now with tissue layer 152 sandwiched between distal layer 102 and proximal layer 104. When this operation is repeated to the opposite side 216, the result is an opening of incision 202 as depicted in FIG. 3D. Tension in the incision indicated by arrows 350 causes' incision edges 352 and 354 to slide proximally to tissue scaffold layer edges 356 and 358, respectively. Now slit 130 can be retracted open without damage to tissue layer 152, or additional slits 360 may be made in closure prosthetic 100 to accomplish the same. It should be understood that a variety of retraction options are known in the surgical art, including placing a dilator in slit 130 or trocar. The essential feature is that even when slits 360 are placed in closure prosthetic 100 it is sufficiently elastic to naturally return to a pre-dilated state when the retraction means is removed. Accordingly, any edges created or provided in closure prosthetic 100 come back in juxtaposition, and the tissue scaffold layer provided in the region of said edges is sufficient to conduct a cellular response that result in these edges becoming fluidically sealed within hours. Additionally, the elasticity of the closure prosthetic can be such that a fluidic seal is established immediately upon removal of the retraction means. Furthermore, it is intended that the anti-adhesive surfaces of distal layer 102 and proximal layer 104 come back into juxtaposition such that the anti-adhesive effect of these layers is not compromised.

As will be apparent to those skilled in the surgical arts in view of this disclosure, attachment means 108 may not always be essential to the optimal working of the closure prosthetic.

### Closure Prosthetic, Example 2

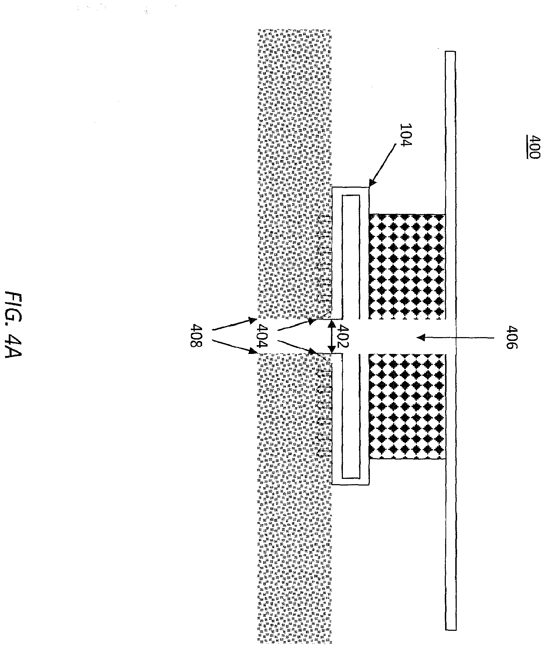

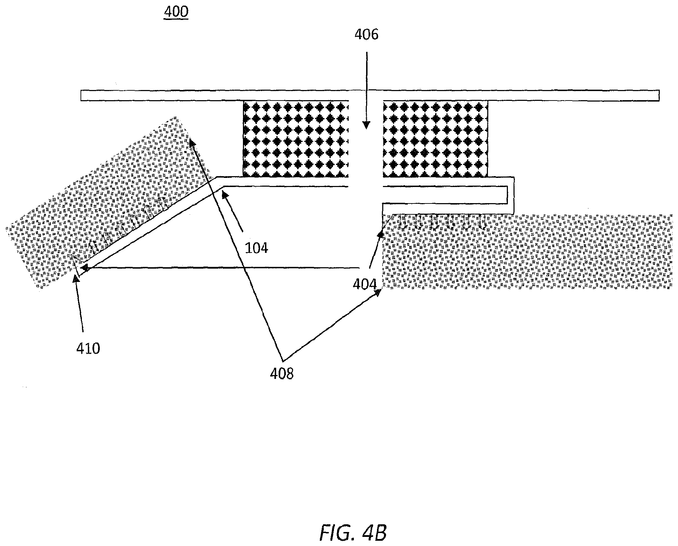

Referring now to FIGS. 4A and 4B, a closure prosthetic 400 is shown with pre-folded proximal layer 104 in FIG. 4A. Edges 402 possess a proximally directed lip 404, as illustrated in FIG. 4B. Between edges 402 is defined the locus of incision such that when incision 406 is made, incision edges 408 naturally open and pass beyond lips 404 to position 410. Now when a dilator (not pictured) is placed in incision 406 proximal layers 104 unfolds. The fold in proximal layer 104 can be manufactured such that it is bistable, and can reside in either the fully folded position or the fully open position such that no matter the degree of dilation of incision 406 proximal layer 104 of closure prosthetic 400 becomes fully open.

Alternatively, the substance of proximal layer can be hydrophilic and induced to swell when in contact with bodily fluids such that shortly after incision 406 is made the swelling of proximal layer 104 causes it to assume the fully open position.

### Closure Prosthetic, Example 3

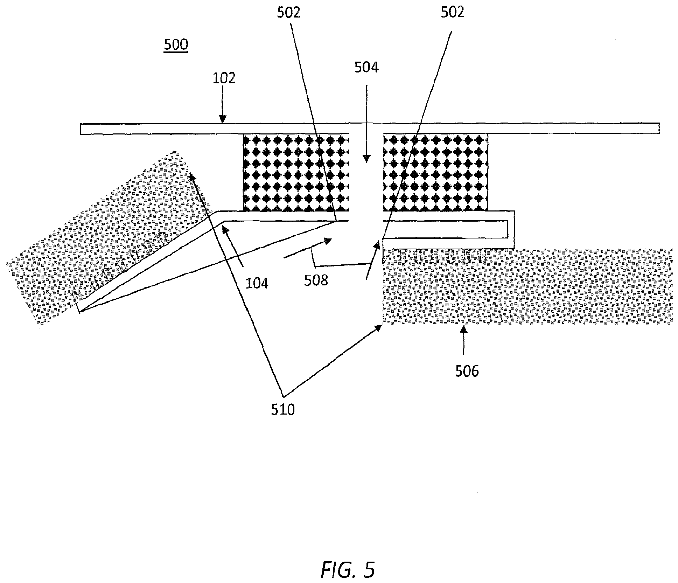

Referring now to FIG. 5, a closure prosthetic 500 is illustrated that includes inversion pulls 502. An incision 504 is made in tissue layer 506 and incision 504 naturally opens. Proximal layer 104 is naturally in the open position. To cause it to fold toward the incision opening, pulls 502 are pulled in direction 508. Proximal layer edges 508 engage incision edges 510 and slide proximally around them, causing tissue layer 506 to be sandwiched between distal layer 102 and proximal layer 104.

### Closure Prosthetic, Example 4

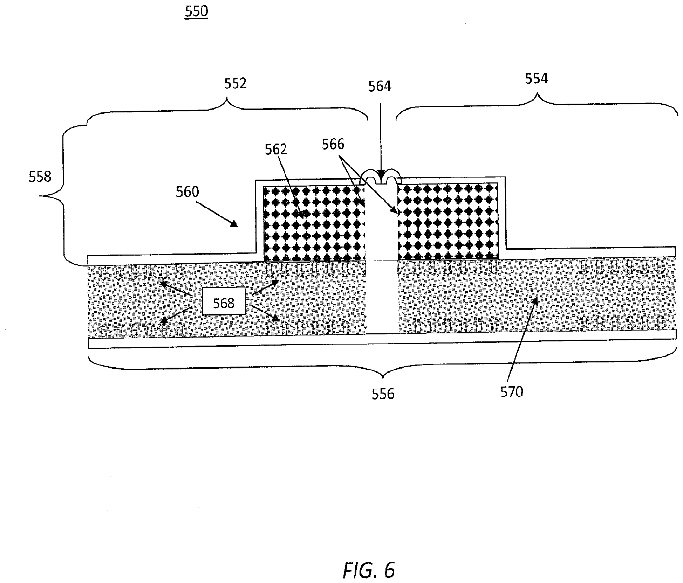

Referring now to FIG. 6, a device and method are illustrated for open heart procedures requiring removal of the parietal pericardium. Generally, this example applies to large incision surgeries. Closure prosthetic 550 is comprised of three parts: left 552 and right 554 distal layers and proximal layer 556. Left and right distal layers are packaged as a single unit 558. Distal unit 558 is comprised on an anti-adhesion sheet 560 bonded to a tissue scaffold layer 562 and divided into two halves. On the distal side of unit 558 are disposed attachment means 564. The two halves of 558 are joined by an interface margin 566. Interface margin 566 is comprised of two parts joined together by a sealing means 564. The sealing means 564 may be hook-and-loop, tacky adhesive, or simply a pair of reinforced strips designed to be sutured together but temporarily adhered together as a packaged unit.

Unit 558 is placed on a tissue layer 570, in this example the parietal pericardium. Doing so engages the attachment means 568. To access the underlying tissue the interface margin 564 is released, and tension in the tissue layer will cause the two halves of unit 558 to part. A tissue pick is used to grasp the pericardium and lift it off the surface of the heart. A scalpel is caused to pierce the pericardium and an incision is run approximately parallel to the open interface margin 564.

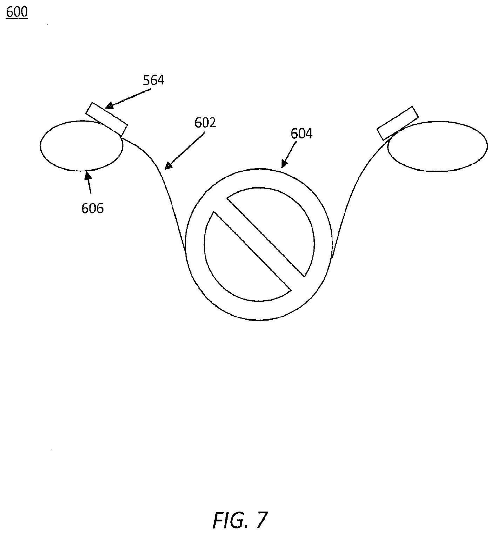

Referring now to FIG. 7, 600 illustrates a transectional view of the pericardium 602 in a sternotomy procedure cradling the heart 604. The two parts of the interface margin 564 are sutured to the sternum 606 in a sternotomy presentation.

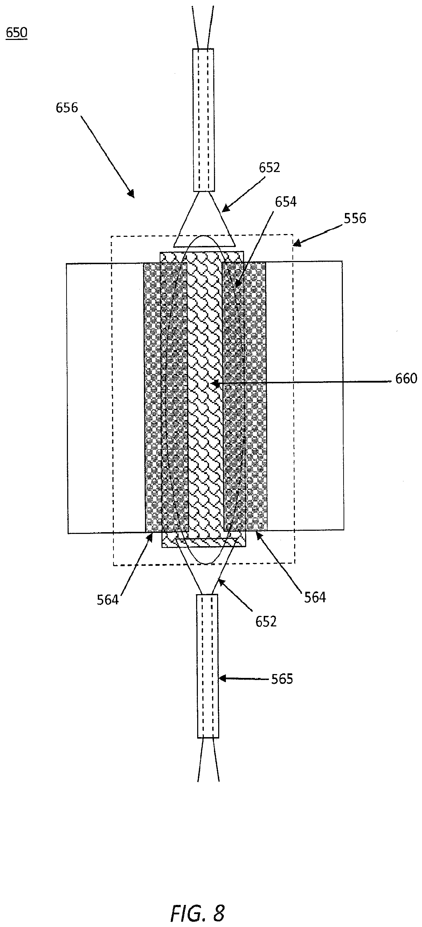

At the conclusion of the open heart surgery it is desirable to close the parietal pericardium around the heart. Care must be taken not to apply pressure to the heart. For this reason, an extender (not shown) may be used to bridge the two parts of the interface margin 564. Referring to FIG. 8, 650 illustrates the closing of the pericardium 656. Two stay sutures 652 are placed at the top and bottom edges of the interface margin 564, the interface margin 564 is brought together by tightening the sutures either by snare 565 or running a knot. When the interface margins 564 are at a suitable distance apart, but not closely in juxtaposition, the proximal anti-adhesion layer 556 is rolled and inserted through the pericardial incision 654 and unrolled between the pericardium 656 and heart. The proximal anti-adhesion layer 556 may also contain a tissue scaffold lamination 660. The proximal anti-adhesion layer is roughly centered on the pericardial incision 654, and if the tissue scaffold lamination 660 is present it is aligned with the tissue scaffold layer 562 of distal unit 558. In this case, when tissue ingrowth occurs in the tissue scaffold layers 660 and 562, they grow together. After this placement, the margins of 564 are drawn together by tightening the sutures 652.

### Closure Prosthetic, Example 5

In some cases the anti-adhesive nature of the closure prosthetic is less important than the tissue conductive aspect. In particular is disclosed the case of aortic aneurysm repair. An aneurysm of a vessel is a localized increase in the diameter of the vessel accompanied by wall thinning. Some strategies in the art concentrate on preventing diameter change in such a vessel, but diameter change is a symptom not a cause and such repairs rarely contain the spread of the aneurysm along the length of the vessel. The cause of the aneurysm is stress in the vessel wall. Although for a sphere, there is less stress in a wall of smaller radius of curvature than one of larger radius of curvature for the same internal pressure, vessels are not spheres. Since vessels are tubes there is a radial pressure as well as axial stress. A vessel can increase its volume either by increasing its diameter or increasing its length, in both cases for fixed initial wall thickness the result in thinning of the vessel wall. Thus simply encircling a bulging vessel does not prevent wall thinning in regions not supported by the encirclement because the encirclement counter acts only the radial pressure and not the axial stress. Clinically this is manifest as a propagation of the bulge beyond any localized encirclement of the vessel. To prevent this from happening, the axial stress must be supported. To achieve this, the encirclement must be affixed to the vessel wall at points beyond the aneurysm so that axial stress cannot cause the vessel wall to slide relative to the encirclement and thus decrease in thickness. One way to achieve this result is to cause tissue to grow into the encircling prosthetic.

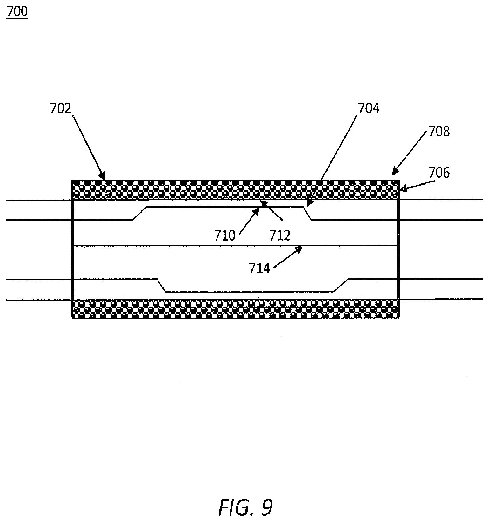

Referring now to FIG. 9, 700 is a closure prosthetic 702 engaged in correcting a vascular aneurysm 704. This is a two layer device comprised of one side of porous tissue scaffold 706 and the other side anti-adhesion layer 708. Such a device is effective for any aneurysm, and is not limited to tubular defects. The porous portion may be absorbable or permanent, and may be reinforced with strengthening members such as mesh, flock, or spun filaments. Axial sliding of the vessel wall 710 relative to a point 712 on the prosthetic 702 is prevented by growth between the vessel wall 710 and prosthetic 702. The shape of closure prosthetic 702 may be a cylinder with a slit 714 cut along its length so that it may be opened and placed around a vessel. Alternatively, prosthetic 702 may be planar, and forced into the shape of a cylinder by sutures placed along its margin. These techniques of attaching a prosthetic to a vessel are well known in the surgical art.

### Closure Prosthetic, Example 6

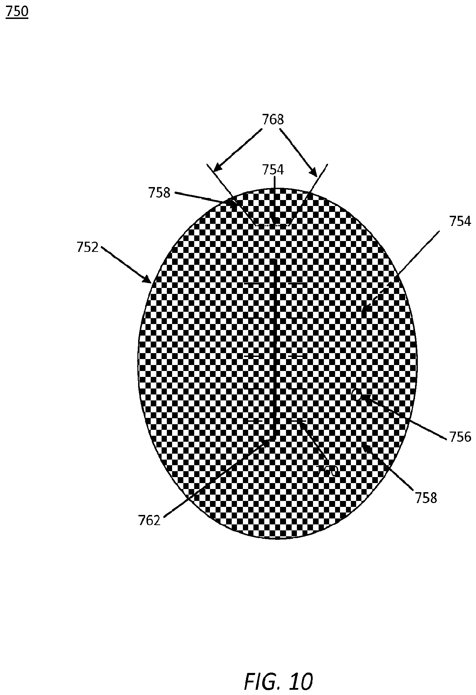

Referring now to FIG. 10, 750 closure prosthetic for the repair of a heart atrium. The atrium is relatively thin-wailed and is frequently opened to perform modifications or replacement of a heart valve. The atrium is primarily a reservoir to the ventricle, and collects blood in a static state prior to filling the ventricle. Blood is particularly susceptible to coagulation when in a static state. It is therefore important that the atrium be closed to prevent blood loss in a manner that does not generate thrombus. One important consideration in this procedure is preserving the alignment of tissue. If the edges of an incision made in the atrium are not aligned to their pre-incision association during closure one side of the incision will end up with more length than the other creating folds. Folds and pockets of any type are particularly thrombogenic, especially if a foreign body such as a suture is also locally present. Therefore, our principle goal in closing the atrium is to preserve tissue alignment and secondarily to prevent adhesion formation between the atrial incision and surrounding tissue.

A single layer closure prosthetic 752 is used, On the proximal side 754 attachment means 756 cover the entire surface. The distal side 758 is entirely smooth and ornamented with lines 760. Closure prosthetic 752 is ideally a circle, but could also be rectangular. When placing closure prosthetic 752 the lines 760 are oriented approximately orthogonal to the intended incision line 762. Light pressure is sufficient to affix closure prosthetic 752 to the atrium surface. Closure prosthetic 752 may additionally possess an incision slit 762, which then is oriented with the intended incision line. In the absence of this feature, closure prosthetic 752 comes with a suture 768 originating of the distal side 758 passing around the proximal side 754 and returning to the distal side 758. In this configuration, closure prosthetic 752 is attached to the atrium and suture 768 is manually pulled radially away from the atrium 764 causing closure prosthetic 752 to lift in the direction of the pulling force. In this configuration, an incision 762 can be made through closure prosthetic 752 and simultaneously through the atrium wall 764. Alternatively, closure prosthetic 752 contains slit 762, and is placed on the atrium. A grasper is used to lift one side of slit 762, which is affixed to atrium 764 and an incision 762 is made in the atrium 764 thus exposed. In both cases, lines 760 ornamenting the closure prosthetic 752 indicate proper alignment of incised closure prosthetic edges 768. When the surgeon is ready to close incision 762 sutures 770 can be placed to close the incision 762 or invert the edges of the incision 762.

### Closure Prosthetic, Example 7

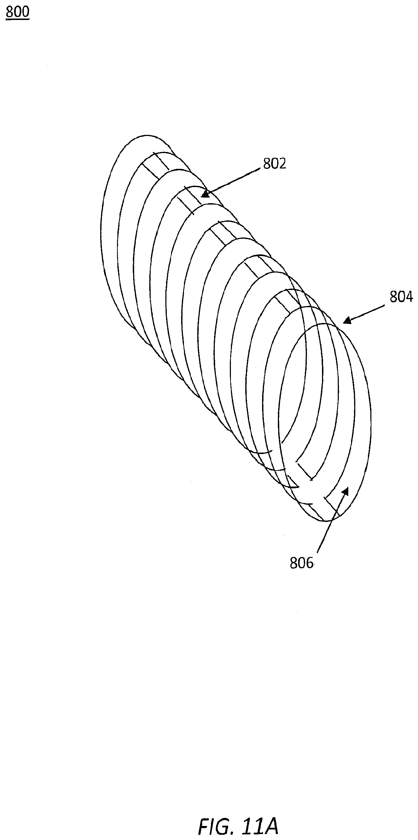

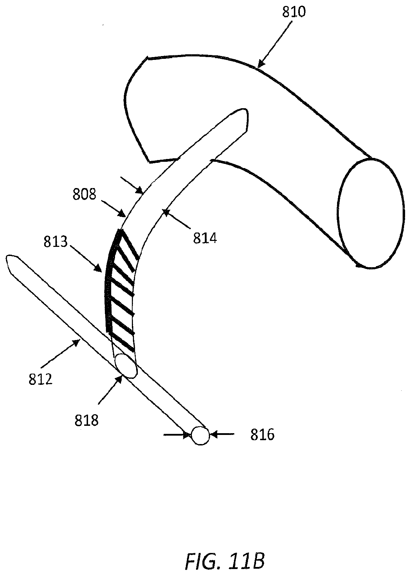

Referring now to FIG. 11A, a closure prosthetic for shielding bypass grafts from adhesions 800 is illustrated. Closure prosthetic 802 can be pre-formed as a cylinder of various diameters or planar. Preferably, it is a ribbed device which can be released around a bypass graft. Closure prosthetic 802 is comprised of distal anti-adhesion layer 804 and proximal tissue scaffold layer 806. Proximal tissue scaffold layer 806 can be absorbable or permanent, while distal anti-adhesion layer is preferably absorbable. It is a particular object of the present invention to provide features for treatment of bypass grafts which are typically of a different diameter and elasticity than the cardiac arteries to which they are attached. Not only does such an encircling prosthetic need to resist adhesion between the bypass graft and the heart and pericardium, but beneficially it can also serve to better match the elasticity of the graft to the cardiac artery. In particular such a prosthetic prevents the dilation of the graft which can create stagnant flows and stresses at the anastomoses site. Referring now to FIG. 11B, bypass graft 808 shunts blood flow from the aorta 810 to a cardiac artery 812. Prosthetic 813 encircles and bends with graft 808. The diameter 814 of the graft is generally considerably larger than the diameter of the cardiac artery 816. Additionally, cardiac artery 812 is generally encased in tissue, and thus resists dilation under pressure. Since the wall stress increases for increasing diameter at constant blood pressure, a larger diameter vessel such as graft 808 will dilate more than the cardiac artery 812. A mismatch in diameters can result in stagnation at the anastomoses 818. Therefore, Example 7 is a treatment for the prevention of graft dilation when pressurized with blood. This is accomplished first by the encircling aspect of closure prosthetic 802, and later by ingrowth between graft 808 and proximal tissue scaffold layer 806. One difficulty in re-operation of patients that have undergone bypass graft procedures is that the graft frequently fibroses to the heart and other surrounding tissues. It would be beneficial if graft 808 remained free from such adhesions. Accordingly, distal layer 804 is provided to prevent such adhesions. Since adhesions typically form within 7 days, the permanence of the anti-adhesion layer is likely not a requisite for clinical success.

### Closure Prosthetic Anti-Adhesion Layer, Example 8

Examples 1-7 can be achieved with the use of various absorbable polymers. The preferred absorbable is a polyurethane.

A bioabsorbable polyurethane can be made by reacting a biocompatible and biodegradable polyisocyanate in the following mixture. The polyisocyanate is preferably a diisocyanate derived from a polypeptide. The polyol component is an aliphatic polyether glycol combined with an absorbable elastomeric polymer derived from the reaction between alkylene glycol and a cyclic bioabsorbable monomer. Common cyclic bioabsorbable monomers are trimethylene carbonate, dioxanone, and caprolactone. The ethylene glycol is preferably a block polymer of polyethylene glycol and polypropylene glycol

Alternatively the bioabsorbable polyurethane can be made by reacting a nonbiodegradable polyisocyanate with a bioabsorbable backbone. The bioabsorbable backbone is comprised of diols and triols selected from the following: hydroxyl-terminated polyethers such as dihydroxy polymers of oxyethylene, ethylene oxide, 1,2-propylene oxide, 1,3-trimethylene oxide, 1,4-tetramethylene oxide, methylene-oxy-1,2-ethylene oxide with molecular weights of 1000 to 10,000 Dalton; polyanhydrides of dicarboxylic acids such as malonic acid, succinic acid, glutaric acid; alcohols such as glycol, 1,2-propylene glycol, 1,3-propylene glycol, butanediol, pentanediol, hexanediol, trimethylolpropane, triethanolamine, pentaerythritol, 2,2-bis(hydroxymethyl)propanol; amino acids such as triols of tyrosine, serine, threonine, cysteine, and sugars such as sorbitol.

Suitable polyisocyanates include: hexamethylene diisocyanate, cyclohexylene diisocyanate, isophorone diisocyanate, p-phenylene diisocyanate, toluene diisocyanate, and 4,4-diphenylmethane diisocyanate.

Dihydroxy polyesters are particularly useful as polyols in absorbable polyurethane compositions. They include polymers of: caprolactone diol, D,L-lactide, D-lactide, L-lactide, glycolide, hydroxybutyrate. Other suitable polyols include copolymers of polyether segments and polyester segments.

Additional synthetic, biodegradable polymeric systems include: poly(amides) such as poly(amino acids) and poly(peptides); poly(esters) such as poly(lactic acid), poly(glycolic acid), poly(lactic-co-glycolic acid), and poly(caprolactone); poly(anhydrides); poly(orthoesters); poly(carbonates); and chemical derivatives thereof e.g., substitutions, additions of chemical groups, for example, alkyl, alkylene, hydroxylations, oxidations, and other modifications routinely made by those skilled in the art, copolymers and mixtures thereof. Representative synthetic, non-degradable polymerizing systems useful in construction of tissue scaffolds include: poly(ethers) such as poly(ethylene oxide), poly(ethylene glycol), copolymers of these and poly(tetramethylene oxide); vinyl polymers⁻⁻ poly(acrylates) and poly(methacrylates) such as methyl, ethyl, other alkyl, hydroxyethyl meihacrylate, acrylic and methacrylic acids, and others such as poly(vinyl alcohol), poly(vinyl pyrolidone), and poly(vinyl acetate); poly(urethanes): cellulose and its derivatives such as alkyl, hydroxyalkyl, ethers, esters, nitrocellulose, and various cellulose acetates: poly(siloxanes); and any chemical derivatives thereof e.g., substitutions, additions of chemical groups, for example, alkyl, alkylene, hydroxylations, oxidations, and other modifications routinely made by those skilled in the art, copolymers and mixtures thereof.

More specifically, the anti-adhesion layers used in constructing the devices and practicing the methods of the present invention can include polylactide polymers and/or copolymers. The resorbable anti-adhesion layer of the present invention is preferably smooth and non-porous. Moreover, the anti-adhesion layer is preferably bioabsorbable in the body, in one embodiment, the anti-adhesion layer material comprises about 60% to about 80% of a polylactide polymer, and about 20% to about 40% of a co-polymer. For example, a healing membrane may comprise poly (L-lactide-co-D,L-lactide) 70:30 Resomer LR708 manufactured and supplied from Boehringer Ingelheim KG of Germany. In one embodiment, the healing membrane has an intrinsic viscosity of about 3 to about 7, preferably about 3.5. In another embodiment, the polylactide has additional copolymers of poly caprolactone or trimethylene carbonate to increase the compliance or flexibility of the film. In this embodiment, the healing membrane material can comprise about 60% to about 80% of a polylactide polymer, and about 20% to about 40% of the co-polymer (caprolactone and/or trimethylene carbonate).

A pre-formed anti-adhesion layer made from the material can be shaped at the time of surgery by bringing the material to its glass transition temperature, using heating iron, hot air, heated sponge or hot water bath methods. The scar-tissue reduction anti-adhesion layer of the present invention preferably has a uniform thickness of less than about 300 microns, preferably less than 200 microns, and more preferably between 10 microns and 100 microns. As defined herein, the "anti-adhesion layer" of the present invention comprise thicknesses between 10 microns and 300 microns and, preferably, between 10 and 100 microns.

In one embodiment, the anti-adhesion layers of the present invention are impregnated with a therapeutically effective amount of an anti-scar forming agent. Angiotensin antagonists have been found to reduce tissue scar formation. See, for example, U.S. Patent No. 6,211.217. Thus, in a preferred embodiment, the healing membranes are impregnated with an angiotensin antagonist. Without wishing to limit the invention to any theory or operation, it is believed that the present healing membrane resorbs and advantageously releases the anti-scar forming agent into the surrounding tissue.

The very thin construction of these anti-adhesion layers is believed to substantially accelerate the rate of absorption of the implants, compared to rates of absorption of thicker anti-adhesion layer implants of the same material. It is believed, however, that resorption into the body too quickly of the anti-adhesion layer will yield undesirable drops in local pH levels, thus introducing/elevating, for example, local inflammation, discomfort and/or foreign antibody responses. Further, a resulting uneven (e.g., cracked, broken, roughened or flaked) surface of a healing membrane degrading too early may undesirably cause tissue turbulence between the tissues before, for example, adequate healing has occurred, resulting in potential tissue inflammation and scarring. It is believed that an anti-adhesion layer of the present invention having a thickness of about 200 microns or less should maintain its structural integrity for a period in excess of three weeks and, more preferably for at least 7 weeks, before substantially degrading, so that the anti-scarring function can be achieved and optimized. To the extent the anti-adhesion layer does not degrade at an accelerated rate, compared to a thicker anti-adhesion layer of the same material, the anti-adhesion layer should maintain its structural integrity for a period in excess of 6 months and, more preferably for at least one year, before substantially degrading, in order to achieve and optimize the anti-scarring function. The polylactide resorbable polymer healing membranes in accordance with this aspect of the present invention are thus designed to resorb into the body at a relatively slow rate.

As used herein, the term "non-porous" refers to a material which is generally water tight and, in accordance with a preferred embodiment, not fluid permeable. However, in a modified embodiment of the invention the anti-adhesion layer may be comprised of two distinct absorbable polymers that are not readily miscible in the melt or solution states. Layers can be formed by melt preparations, either molded or extruded or by solution preparations using a casting methodology. In either case, once the layers are formed and still in their fluid state, the two polymeric components will tend to separate, creating micelles of predominately one component or the other. If these two absorbable polymeric components have different absorption rates in the body, then at some point within the body the anti-adhesion layer will become porous due to the earlier resorption of one of the polymeric components. The primary object of this type of composition is to choose the faster resorbing component to first pass into a gel phase before it is absorbed. The value of this construction is to create an anti-adhesion layer that is self-lubricating as the faster resorbing component is released from the layer and spreads across the layer. This may aid in the maturation of the tissue scaffold layer because micro-pores may exist in the resorbable anti-adhesion layer of the present invention, to the extent, for example, that they do not substantially disrupt the smoothness of the surfaces of the resorbable anti-adhesion layer to cause scarring of tissue. In substantially modified embodiments for limited applications, pores which are cell permeable but not blood vessel permeable may be manufactured and used.

### Closure Prosthetic Tissue Scaffold Layer. Example 9

The scaffold layer is preferably porous, and more preferably open-cell. Alternatively, the scaffold layer may be absorbable with a cell attachment compound such as lysine so that cells populate the surface of the scaffold layer and tissue structures are constructed to replace degradation of this layer.

Preferably, the scaffold later is an absorbable polyurethane, but it may be any absorbable composition as detailed in Example 8. There are many methods known in the art for constructing porous or foamed polyurethane. The porosity is generally generated during the polymerization process which yields the polyurethane. This is especially true for cross linked polyurethanes, which cannot be placed in a liquid state by solvents or heat once they are formed. The methods include dissolving under pressure inert gases in the prepolymer, evaporating volatile inert liquids during polymerization, inserting compounds that decompose into gas when subjected to the heat released during polymerization, adding water which reacts with isocyanate groups to form amine and carbon dioxide, adding solid phase particulate that can be dissolved after polymerization, solution polymerization under phase separation, and mechanically admixing gases.

A characteristic of polymerized polyurethane compositions is that the pores tend to close on the surface due to surface tension. Open cell layers can be obtained by mechanically removing this layer. However, this process is cumbersome and polymerizing the scaffold layer on at least one of the anti-adhesion layers is preferred since it will advantageously bond to this layer.

Open surface cells can be obtained by additives. Surfactants are useful, but need to be removed before implantation. Nucleating agents such as inert particulate are useful for uniform cell size and distribution.

The addition of monosaccharides, disaccharides, oligosaccharides and poly saccharides improves uniformity of porosity and open surface cells. The amount needed is typically less than 4% by weight. Preferred saccharides for use in obtaining open surface cells are monosaccharides, such as dextrose, mannose, mannitol, dulcitol, glucose, fructose, galactose; disaccharides such as maltose, lactose, saccharose, cellobiose; and oligosaccharides and polysaccharides such as cellulose, pectin, amylopectin.

Open porosity on the surface is insufficient if the internal porosity is not interconnected. The object of a tissue construct is to provide for tissue and vascular infiltration throughout the scaffold. In order to obtain interconnected porous networks inside the tissue scaffold layer an elongate nucleation additive is placed in the prepolymer preparation prior to curing. Filaments, or similar structures will aggregate gas phase carbon dioxide or other blowing gases along their length. If the density of filaments is sufficient, then surface tension will cause the aggregation of many bubbles to coalesce forming interconnected elongate pore formation.

### Mechanical Properties of the Closure Prosthetic, Example 10

The closure prosthetic of the present invention promotes healing of damaged tissue after surgery, the device comprising: an anti-adhesive layer joined to a tissue scaffold layer, the resulting device is: (a) substantially-smooth on one external side; (b) substantially porous on another external side; (c) constructed of a resorbable polymer on the smooth side; and (d) adapted to conduct cells and vascular structures through the tissue scaffold layer after implantation of the prosthetic into the mammalian body.

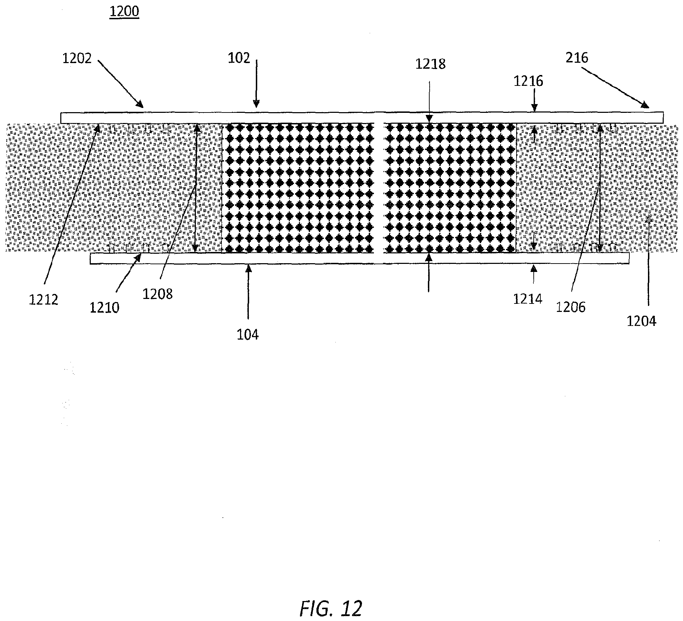

Referring now to FIG. 12, a three layer closure prosthetic, of the present invention 1202, for repair of the parietal pericardium of a human 1204 is illustrated for the purposes of specifying dimensional parameters. Human pericardium possesses a mean thickness 1206 on the ventral side of approximately 360 um. A preferred embodiment would simulate the dimensional and mechanical characteristics of the native pericardium. The distal anti-adhesion layer 102 is attached to the proximal adhesion layer 104 by a sandwiched and bonded tissue scaffold layer 106. Distance 1208 is the length from the distal surface 1210 of the proximal layer 104 to the proximal surface 1212 of the distal layer 106. This distance is between 100 um to 1000 um, and preferably between 300 um and 400 um and most preferably 360 um. Thickness 1214 of proximal layer 104 and the thickness 1216 of distal layer 106 are between 50 um and 500 um, and preferably between 100 and 300 um. It follows thickness 1218 of the scaffold layer is equal to distance 1208.

The tensile strength of human pericardium is between 1.1 and 11.4 MPa. However, a stress of less than 100 kPa will constrict ventricular filling, and result in reduced cardiac output. Therefore, as a safety feature, one would prefer the closure prosthetic to fail rather than constrict the heart. The tensile strength is relatively unimportant given the highly elastic nature of the pericardium. The preferred mechanical property is that the closure prosthetic of the present invention has a maximum stress/strain relation approximately equal to that of the native human parietal pericardium. Consequently, a preferred elongation at break would be approximately 1000%. This is achievable for a variety of polyurethanes.

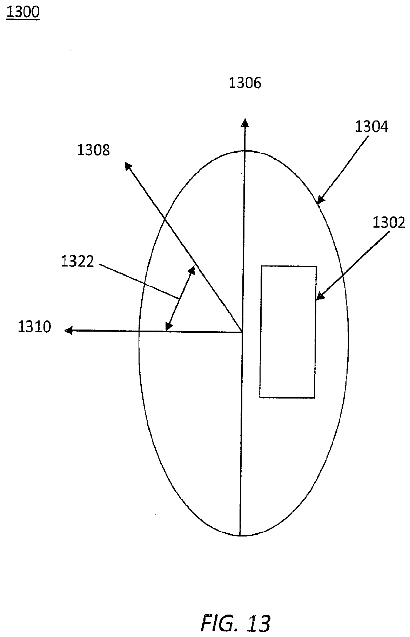

The stresses placed on the pericardium are primarily due to the motions of the heart. The heart contracts in a spiral motion about the apical axis. The spiral angle is approximately 30 degrees from this axis. In addition, most pathologies of the heart modify the shape of the heart from a cone or cylinder to a sphere. Spherical morphologies are energy inefficient, distort the valve plane, and create pathological stress in the ventricular free walls. There is a benefit to restoring the heart to a cylindrical geometry. Therefore referring now to FIG. 13, 1300 illustrates the closure prosthetic 1302 of the present invention relative to its placement on a human heart 1304. The apical axis of the heart 1306, direction of contraction of the heart 1308, circumferential direction 1310 and spiral angle 1312 are illustrated. Preferably the closure prosthetic 1302 has a higher modulus in the circumferential direction 1310 than in the contraction direction 1308. This choice of moduli will tend to increase heart size in the apical axis 1306 and restrict heart size in the circumferential axis 1310. Preferably, the ratio of the moduli, modulus(circumference)/modulus(contraction), is approximately 2. Accordingly, a preferred embodiment of the present invention is a pericardial closure prosthetic with a modulus anisotropy of 2 and an angle of 30 degrees where the primary modulus axes are indicated on the prosthetic for orientation during placement in a surgical procedure.

In the placement of the closure prosthetic of the present invention, the device should be placed to minimize stress in the pericardium. Although attempts have been made to forcibly constrain the heart with various ventricular wraps and surgical remodeling procedures, these efforts have been generally unsuccessful. However, a prosthetic of the present invention could be employed in this type of surgical procedure, It is anticipated, however, that the closure prosthetic will be placed with a minimum amount of tension in the parietal pericardium. One of the advantageous aspects of an anisotropic prosthetic is that if subsequent to the pericardial closure procedure the heart should suffer congestion and become hypertrophic, the prosthetic should help to direct the increased volume of the pathologic heart into a preferred cylindrical or more precisely conical geometry while retaining near-normal compliance.

### Fixation Design of the Closure Prosthetic, Example 11

A closure device of the present invention in some cases will require localization to the repair site. Conventional fixation means such as sutures, tacks, staples and the like may be used as well as various surgical sealants and adhesives, In some embodiments of the present invention a proximal layer lies opposite a distal layer, and it may not be desirable to couple the proximal layer and distal layer by any means other than through the tissue scaffold layer provided. In particular, it is generally undesirable for the fixation means to apply pressure to tissue sandwiched between the proximal and distal layers, as would happen if for example sutures were used. It is a particular novelty of the present invention that the proximal and distal layers have independent fixation means, and that the fixation means resides on the closure prosthetic. It is a further advantageous feature that the fixation means engages tissue when this tissue is properly located in the device independently of any specific actions by the surgeon. Furthermore, the fixation means is selectively engaging, allowing tissue to enter the space between said proximal and distal layers without incumberance, but resisting removal of tissue once this space is created around a tissue layer or entered by the tissue. Lastly, the absence of pressure applied to peripheral wound tissue by the closure prosthetic preserves the viability of this tissue by avoiding ischemia and pressure necrosis.

The present invention achieves fixation by the exposure of repair-site tissue to engaging elements located in the space between the proximal and distal layers of the closure prosthetic. Tissue exposure can be achieved by reorienting, as through folding, one or more layers so as to engage tissue, provide the engagement means with a preferred direction, or envelope the engagement means in a water soluble material which then exposes the engagement means upon contact with fluids in the operative environment.

While each exposure means is suited to particular surgical scenarios, the one anticipated to be of greatest general utility, especially when the closure prosthetic contains at least two anti-adhesion layers, are the directed engagement elements. The elements are preferably numerous so that any one element bears a small portion of the total fixation force and the elements themselves penetrate only superficially a tissue lay er. The superficial penetration of the engagement elements is beneficial because this design avoids adverse effects associated with sutures, stables, and tacks, e.g. the entrapment of blood vessels and nerves.

The range of force applied by the closure prosthetic for fixation can be controlled to some extent by the geometrical shape of the engagement methods. The engagement elements are fashioned from the material of the respective layer and are integral to this layer. Correspondingly, if the layer is absorbable, then, too. the fixation elements will lose their fixative potential with time. This is a beneficial feature since the purpose of the tissue scaffold layer is to eventually be replaced with viable tissue and the anti-adhesion layers leave the body, in many cases, but not the pericardial repair case, merely a roughened surface is sufficient. A sufficiently rough surface may be one with grooves, bumps, or protruding filaments which at first mechanically resist motion of the closure prosthetic, and subsequently induce fibrosus or denaturation of aqueous proteins which act as a natural adhesive.

In more demanding situations, where a tissue layer possesses a defect and there exist forces present that resist closure of the defect, such as is the case with an incised parietal pericardium, a more tenacious engagement element is required. Preferred geometries mimic those found in nature, such as the seed pods of Xanthium strumarium, the common cocklebur plant. The burs of this plant are covered with thousands of stiff, hooked spines. Man-made equivalents follow a hook-and-loop design. Whether the engaging element is simply recurved, flexible or stiff, or tipped with a barb the important design feature is that they be numerous and of small dimension relative to the tissue thickness.

## Claims

1. A prosthetic for promoting healing of damaged tissue after an open heart surgery, the prosthetic comprising a fluidically sealing membrane, which includes (a) a substantially-smooth first absorbable layer (102), (b) a substantially-smooth third absorbable layer (104), and (c) a porous second layer (106) binding together said first and third layers to provide a scaffold for tissue regeneration, **characterized in that** the membrane is adapted so that said first and third layers are resorbed into the mammalian body after said second layer is ingrown with tissue forming a fluidically sealed layer of living tissue, wherein said second layer is smaller in area than said first and third layers such that first and third layers are not bound together at the periphery.

2. A prosthetic of claim 1, wherein said first and third layers are resorbed within 6 to 18 months and said second layer is resorbed after said first and third layers are resorbed.

3. The prosthetic of claim 1, wherein the step of placing the prosthetic in a patient is effective to attenuate tissue adhesion between the epicardium and the prosthetic and between external tissue and the prosthetic.

4. The prosthetic of claim 1, wherein the first and third layers are left unbound over an area sufficient to allow a patient's tissue to be placed between said first and third layers and sandwiched therein using a method of fixation sufficient to create a fluidic seal around the periphery of the prosthetic, and/or
wherein said first layer is smooth on the side facing the or a patient's epicardium and rough on the side facing away from said patient's epicardium; said third layer is smooth on the side facing a patient's non-cardiac tissue and rough on the side facing toward said patient's heart such that the pocket formed by first and third layers grasps and retains tissue, when said patient's tissue is introduced in the space created between said first and third layers, preferably wherein said rough sides of said first and third layers are provided with hooking structures sufficient to retain tissue.

5. The prosthetic of claim 1, wherein the periphery is reinforced and of sufficient tear strength to retain sutures.

## Patentansprüche

1. Prothese zum Fördern einer Heilung von beschädigtem Gewebe nach einer Operation am offenen Herzen, die Prothese umfassend eine fluidisch abdichtende Membran, die (a) eine im Wesentlichen glatte erste absorbierbare Schicht (102), (b) eine im Wesentlichen glatte dritte absorbierbare Schicht (104) und (c) eine poröse zweite Schicht (106), die die erste und die dritte Schicht miteinander verbindet, um eine Struktur für eine Geweberegeneration bereitzustellen, beinhaltet, **dadurch gekennzeichnet, dass** die Membran so angepasst ist, dass die erste und die dritte Schicht in den Säugetierkörper resorbiert werden, nachdem die zweite Schicht mit Gewebe verwachsen ist, das eine fluidisch abgedichtete Schicht aus lebendem Gewebe ausbildet, wobei die zweite Schicht flächenmäßig kleiner als die erste und die dritte Schicht ist, derart, dass die erste und die dritte Schicht an dem Umfang nicht miteinander verbunden sind.

2. Prothese nach Anspruch 1, wobei die erste und die dritte Schicht innerhalb von 6 bis 18 Monaten resorbiert werden und die zweite Schicht resorbiert wird, nachdem die erste und die dritte Schicht resorbiert wurden.

3. Prothese nach Anspruch 1, wobei der Schritt eines Platzierens der Prothese in einen Patienten effektiv ist, um die Gewebeadhäsion zwischen dem Epikard und der Prothese und zwischen externem Gewebe und der Prothese abzuschwächen.

4. Prothese nach Anspruch 1, wobei die erste und die dritte Schicht über einen Bereich ungebunden bleiben, der ausreicht, um zu ermöglichen, dass das Gewebe eines Patienten zwischen der ersten und der dritten Schicht platziert und unter Verwendung eines Fixierungsverfahrens, das ausreicht, um eine fluidische Abdichtung um den Umfang der Prothese herum zu erzeugen, darin eingebettet wird, und/oder
wobei die erste Schicht auf der Seite, die dem Epikard des Patienten zugewandt ist, glatt und auf der Seite, die von dem Epikard des Patienten abgewandt ist, rau ist; die dritte Schicht auf der Seite, die dem nicht-kardialen Gewebe des Patienten zugewandt ist, glatt ist, und auf der Seite, die dem Herzen des Patienten zugewandt ist, rau ist, derart, dass die Tasche, die durch die erste und die dritte Schicht ausgebildet wird, Gewebe erfasst und hält, wenn das Gewebe des Patienten in den Raum eingeführt wird, der zwischen der ersten und der dritten Schicht ausgebildet wird, vorzugsweise wobei die rauen Seiten der ersten und der dritten Schicht mit Hakenstrukturen versehen sind, die ausreichen, um das Gewebe zu halten.

5. Prothese nach Anspruch 1, wobei der Umfang verstärkt ist und eine ausreichende Reißfestigkeit aufweist, um Nahtmaterial zu halten.

## Revendications

1. Prothèse pour favoriser la guérison d'un tissu endommagé après une chirurgie à cœur ouvert, la prothèse comprenant une membrane d'étanchéité aux fluides, qui comporte (a) une première couche absorbable (102) sensiblement lisse, (b) une troisième couche absorbable (104) sensiblement lisse et (c) une deuxième couche poreuse (106) liant ensemble lesdites première et troisième couches afin de fournir un échafaudage pour la régénération tissulaire, **caractérisée en ce que** la membrane est adaptée de sorte que lesdites première et troisième couches sont résorbées dans le corps de mammifère après que ladite deuxième couche est incarnée avec du tissu formant une couche scellée aux fluides de tissu vivant, ladite deuxième couche ayant une superficie plus petite que lesdites première et troisième couches de sorte que les première et troisième couches ne sont pas liées ensemble à la périphérie.

2. Prothèse selon la revendication 1, dans laquelle lesdites première et troisième couches sont résorbées de 6 à 18 mois et ladite deuxième couche est résorbée après que lesdites première et troisième couches sont résorbées.

3. Prothèse selon la revendication 1, dans laquelle l'étape de placement de la prothèse chez un patient est efficace pour atténuer l'adhérence tissulaire entre l'épicarde et la prothèse et entre le tissu externe et la prothèse.

4. Prothèse selon la revendication 1, dans laquelle les première et troisième couches sont laissées non liées sur une zone suffisante pour permettre au tissu d'un patient d'être placé entre lesdites première et troisième couches et d'être pris en sandwich à l'intérieur à l'aide d'un procédé de fixation suffisant pour créer un joint fluidique autour de la périphérie de la prothèse, et/ou
ladite première couche étant lisse sur le côté faisant face à l'épicarde ou une épicarde d'un patient et rugueuse sur le côté faisant face à l'épicarde dudit patient ; ladite troisième couche étant lisse sur le côté faisant face au tissu non cardiaque d'un patient et rugueuse sur le côté tourné vers le cœur dudit patient de sorte que la poche formée par la première et la troisième couches saisit et retient le tissu, lorsque ledit tissu du patient est introduit dans l'espace créé entre lesdites première et troisième couches, de préférence lesdits côtés rugueux desdites première et troisième couches étant fournis de structures d'accrochage suffisantes pour retenir le tissu.

5. Prothèse selon la revendication 1, dans laquelle la périphérie est renforcée et de résistance à la déchirure suffisante pour retenir les sutures.
